# EUROPÄISCHE PATENTSCHRIFT

(11) **EP 2 703 019 B1**
(45) Veröffentlichungstag und Bekanntmachung des Hinweises auf die Patenterteilung: **18.01.2017**
(21) Anmeldenummer: 13004276.5
(22) Anmeldetag: 30.08.2013
(51) Int. Cl.: A61M 3/02

(54) **Vorrichtung zur Einbringung eines Irrigationsmediums und/oder zur Triggerung eines Defäkationsreflexes im Rahmen einer Wegwerfeinmalanwendung**
Device for introducing an irrigation medium and/or for triggering a defecation reflex as part of a disposable single use application
Dispositif d'introduction d'un moyen d'irrigation et/ou de déclenchement d'un réflexe de défécation dans le cadre d'une utilisation unique jetable

(30) Priorität: 30.08.2012 DE 102012017144
(43) Veröffentlichungstag der Anmeldung: 05.03.2014
(73) Patentinhaber: Advanced Medical Balloons GmbH, 68753 Waghäusel (DE)
(72) Erfinder: Göbel, Fred, 69259 Wilhelmsfeld (DE)
(74) Vertreter: Küchler, Stefan

(56) Entgegenhaltungen:
- EP-A1- 0 799 610
- US-A- 4 563 182
- US-A1- 2010 022 976
- US-B1- 6 575 954

## Beschreibung

Die Erfindung richtet sich auf eine Vorrichtung zum trans-analen Einbringen eines Einlaufs in das Rektum bzw. Colon eines Patienten, umfassend einen aufblasbaren Ballon von durch Präformierung bei der Herstellung vorgegebener, taillierter, insbesondere hantel- oder sanduhrförmiger Gestalt, mit zwei endständigen Ballonabschnitten von größerem Radius und etwa sphärischer oder diskoider Gestalt, sowie einem dazwischen angeordneten, mittigen, verjüngten Ballonabschnitt von verringertem Radius, welcher trans-anal platziert wird, derart, dass der distal anschließende, radial erweiterte Ballonabschnitt intra-rektal platziert ist und der proximal anschließende, radial erweiterte Ballonabschnitt extra-korporal. Insbesondere richtet sich die Erfindung auf eine Vorrichtung zum trans-analen Einbringen eines Einlaufs in das Rektum bzw. Colon eines Patienten, mit einem trans-anal platzierten aufblasbaren Ballon von einer hantel- oder sanduhrförmigen Gestalt, wobei die Ballonenden auf dem Körper des Katheterschaftes derart invertiert positioniert sind, dass sich die endständigen Ballonanteile bei Befüllung über den mittigen, verjüngten Ballonanteil hinweg, in gegengerichteter Weise aufeinander zu bewegen, und die distale Öffnung des Katheterschaftes dabei den Scheitelpunkt des vorderen, frontalen Ballonradius bei axial nicht-ausgelenkter Ruhelage des Schaftkörpers im trans-anal platzierten Ballon nicht nach distal hin überschreitet Katheter zur trans-analen Applikation eines Einlaufes in den Enddarm (Rektum) bzw. den Dickdarm (Colon) eines Patienten sind in verschiedenster Ausführungsform seit längerem bekannt.

Einlaufkatheter können als einfache Schlauchelemente ausgeführt sein, zum verbesserten Halt des Katheters im Rektum aber auch mit einem intra-rektal platzierten Ballonelement versehen werden. Derartige Ballonelemente bieten neben einer ano-rektal ankernden Funktion auch einen gewissen Dichtungseffekt, der die durch die Irrigation eingebrachte Flüssigkeit im Darm hält. Das Dichtungsvermögen von Einlaufkathetern mit ausschließlicher intra-rektaler Ballonkomponente ist in vielen Fällen jedoch nicht ausreichend und muss vom Anwender durch fortwährende manuelle Lagekorrektur der dichtenden Ballonflächen auf dem Rektumboden bzw. Manipulation der Stellung des dem Ballon aufsitzenden Katheterschaftes hergestellt werden.

Ein wesentliches Risiko bei der Anwendung von handelsüblichen, trans-anal in den Darm eingeführten Kathetern besteht nach wie vor in der Perforation der Darmwandung bei unsachgemäßer Handhabung oder auch bei vorgeschädigten bzw. entsprechend geschwächten Darmwandstrukturen. Derartigen Perforationen des Darms kann bislang nur durch eine entsprechende Schulung und Sensibilisierung des Anwenders auf diese besondere Problematik vorgebeugt werden.

Im Verlauf der letzen zehn Jahre hat sich über die Anwendung konventioneller trans-analer Einläufe hinaus eine neuartige Einlauftechnologie etabliert, die durch intermittierende, relativ kleinvolumige intra-rektal eingebrachte Einläufe die reflexkoordinierte aktive Entleerung des Rektums und weiten Anteilen des sich an das Rektum anschließenden linksseitigen Colon auslöst. Durch die konsequente Entleerung dieser Darmabschnitte kann der Patient so, trotz bestehender Inkontinenzproblematik, in den Zustand einer sogenannten "Pseudo-Kontinenz" überführt werden. Das Verfahren der sogenannten trans-analen Irrigation (TAI) kann vom Patienten selbst durchgeführt werden.

Die Funktion des rektal instillierten Einlaufmediums besteht neben einer gewissen Mobilisierung von Stuhl vor allem in einer moderaten Dehnung der Wandanteile der Rektumampulle (terminales Rektum), die in der Folge zur Auslösung des physiologischen Stuhlentleerungsreflexes (Reflextriggerung) führt. Während das rektal platzierte Einlaufmedium in der Regel bereits nach wenigen Minuten eine derartige Reflextriggerung verursacht, kann die sich anschließende vollständige Entleerung des angewendeten Irrigationsvolumens aus dem Rektum bzw. Colon deutlich längere Zeit, bis hin zu 30 Minuten oder darüber hinaus erfordern. Der relativ hohe Zeitbedarf der TAI schränkt die Akzeptanz bei vielen Patienten ein, und schließt die Methode, obwohl beim Einzelnen in der Regel effizient anwendbar, häufig aus.

Problematisch ist zudem, dass viele Anwender nicht in der Lage sind, die Irrigationskatheter konventioneller Bauart manuell in eine ausreichend dichtende Lage zu bringen und zu halten.

Ferner kann es bei der Aufdehnung der Ballonkomponente herkömmlicher Katheter innerhalb des Rektums bereits bei dessen beginnender Füllung zu einer reflextriggernden Dehnung von Darmwandanteilen kommen, die unter anderem zu einer reflexartigen Öffnung des Anus, und so unter Umständen zum Herausgleiten des ankernden Ballons aus dem Rektum führt.

Im Markt verfügbare Vorrichtungen zur intermittierenden trans-analen Irrigation, wie z.B. das System Peristeen - Anal Irrigation, der Fa. Coloplast, Dänemark, sind darüber hinaus relativ aufwendig konstruiert, und oft nur bedingt für die Anwendung außerhalb des vertrauten häuslichen Bereichs geeignet.

Wünschenswert für die verbesserte Akzeptanz der trans-analen Irrigation beim Anwender wäre eine Kathetertechnik, die den Katheterschaft nach dessen Einführen ins Rektum ohne erforderliche fortwährende Lagekorrektur, in seiner trans-analen Position hält, und sowohl das potentiell traumatische Hineingleiten des Schaftkörpers in den Darm verhindert, als auch das verfrühte unerwünschte Herausgleiten des Katheters aus dem Anus zuverlässig vermeidet.

Einen besseren Komfort hinsichtlich der trans-analen Platzierung des Katheters bieten in dieser Hinsicht Einlaufkatheter mit Doppelballonanordnung. Dem Katheterschaft sitzen hier, von einander auf dem Schaft beabstandet, zwei separate, in der Regel über ein einziges Füll-Lumen gleichzeitig befüllte Ballonelemente auf. Diese kommen auf beiden Seiten des Analkanals, also innen und außen, zu liegen bzw. nehmen die Strukturen des Anus zwischen sich auf. Die in der Regel elastische Ausführung des Ballonmaterials führt bei Aufdehnung der Ballonwandung unter Druck zu einer sphärischen Erweiterung der Ballonkörper und so zu einer gewissen axialen wirkenden Quetschung des zwischen den Ballonkörpern liegenden Anus. Die dislokationssichere Positionierung des Katheterschaftes ist bei derartigen Kathetern als relativ sicher anzunehmen. Problematisch sind die zum Teil hohen, zur elastischen Aufdehnung der Hülle erforderlichen Fülldrucke, die von Patienten als störendes oder auch schmerzhaftes Fremdkörpergefühl empfunden werden. Ferner kann die elastische Aufdehnung des intra-rektalen Ballons zu einer prall gefüllten Sphäre zur unmittelbaren Triggerung des Defäkationsreflexes führen, was wiederum bei simultaner rektaler Kontraktion und nachlassendem Schließmuskeltonus im ungünstigsten Fall zum Herausgleiten des Katheters und zur verfrühten Entleerung der Irrigationsflüssigkeit führt.

US 2010/0022976 offenbart eine Vorrichtung zur Verwendung bei rektaler Inkontinenz, wobei die Vorrichtung einen invertiert an einem Katheterschaft befestigten Ballon aufweist. US 4,563,182 beschreibt eine Verpackung für eine Vorrichtung zur Behandlung von Hämorrhoiden.

Die DE 10 2004 033 425 B4 beschreibt ein Verschlußsystem zur Versorgung rektaler bzw. analer Inkontinenz mit einer besonderen Ausführungsform für die Tamponade blutender Hämorrhoiden, wobei ein tailliertes Ballonelement dargestellt ist, welches jeweils endständig über einen intra-rektalen bzw. prä-analen Abschnitt verfügt. Die Ballonhülle ist dabei derart auf dem ballontragenden Schaftkörper platziert, dass sich das intra-rektale Ballonsegment bei Befüllung zum Rektumboden hin bewegt und dort auf die blutenden venösen Gefäße einen tamponierenden Druck ausübt. Entsprechend bewegt sich das prä-anale Ballonsegment zur äußeren Analöffnung hin. So stellt sich eine von beiden Seiten axial auf den Anus gerichtete Tamponadewirkung ein. Zusätzlich zur axialen Blutungstamponade dehnt sich der mittlere taillierte Abschnitt des Ballons radial zur Wandung des Analkanals hin aus. Er ist bevorzugt mit einem Durchmesser ausgeformt, der den Durchmesser des geöffneten Analkanals überschreitet. Die Spitze des Schaftkörpers ragt bei der beschrienen Vorrichtung zur Akutversorgung einer venösen ano-rektalen Blutung im Zustand der Anwendung frei und ungeschützt in den Darmraum, und stellt ein potentielles Verletzungsrisiko dar.

Die WO 2007/118621 A1 beschreibt ein ähnliches hantel- bzw. sanduhrförmiges Ballon-Verschlußsystem. Ihm liegt die Aufgabe zu Grunde, für intermittierende Zeiträume von wenigen Stunden bei chronisch ano-rektal inkontinenten Patienten eine Dichtung gegen unwillkürlich austretenden Stuhl zu gewährleisten. Bei dieser Vorrichtung zur Versorgung steht ebenfalls die von beiden Seiten axial zum Anus hin gerichtete Rollbewegung der endständigen Ballonsegmente im Vordergrund. Auch hier ragt die Spitze des den Ballon tragenden Schaftkörpers im trans-anal platzierten Zustand frei und potentiell traumatisierend in den Darmraum.

Beide Vorrichtungen beschreiben eine sanduhr- bzw. hantelförmige Ballongestalt, wobei der Anus im taillierten, gegenüber den endständigen Portionen des Ballons verjüngten Bereich aufgenommen wird. Durch eine derartige Ausformung des Ballons kann neben einer relativ guten Sicherung des Katheters vor Dislokation, wegen der allseitigen Dichtung der Ballontaille innerhalb des Analkanals auch eine, gegenüber konventionellen Doppelballons deutlich verbesserte Verschlussleistung erreicht werden. Auch eine Irritationen, Schmerzen oder einen Defäkationsreflex auslösende Aufdehnung der Ballonhülle kann bei beiden Vorrichtungen weitgehend vermieden werden, da die Ballonwandung vorzugsweise bereits auf ihr Arbeitsmäß oder darüber hinaus ausgeformt ist. Zur Befüllung bzw. Entfaltung des Ballons in seinen Arbeitszustand ist daher lediglich ein niedriger Fülldruck erforderlich, der dem jeweils im Rektum bzw. im Bauchraum herrschenden Druck entspricht, oder diesen nur gering überschreitet.

Als besonders nachteilig bei den in DE 10 2004 033 425 B4 und WO 2007/118621 A1 beschriebenen Ausführungen erweist sich jedoch die sich bei Befüllung des Katheterballons einstellende frei exponierte distale Spitze des den Ballon tragenden Schaftkörpers, welche in das Darmlumen hineinragt und dort im Rahmen der Anwendung zu entsprechenden Irritationen oder Verletzungen der Darmwandung führen kann.

Das die Erfindung initiierende Problem besteht darin, diese Nachteile des bekannten Standes der Technik zu vermeiden.

Die Lösung dieses Problems gelingt anspruchsgemäß bei einer gattungsgemäßen Anordnung dadurch, dass der Katheterschaft in einer aus Folienlagen verschweißten Folienstruktur verpackt ist, deren ausgeformte Kompartimente derart um den Katheter herum angelegt sind, dass sich eine sinnvolle, über das Produkt hinweg gerichtete lineare Abfolge von Anwendungsschritten ergibt, die mit der Benetzung der Katheterspitze mit einem Gleitmittel beginnt, sich mit der Entnahme des benetzten Katheters aus einem aufreißbaren taschenartigen Kompartiment fortsetzt und mit der Einbringung einer im Produkt anwendungsfertig integrierten Menge an Irrigationsflüssigkeit in das Rektum abschließt.

Eine derartige Vorrichtung erlaubt das trans-anale Einbringen eines Einlaufs in das Rektum bzw. Colon eines Patienten durch eine sinnvolle, über das Produkt hinweg gerichtete, lineare Abfolge der folgenden Anwendungsschritte:
- In einem ersten Schritt wird die Katheterspitze mit einem Gleitmittel benetzt;
- in einem zweiten Schritt wird der benetzte Katheters aus einem aufreißbaren taschenartigen Kompartiment entnommen;
- in einem dritten Schritt wird eine im Produkt anwendungsfertig integrierte Menge an Irrigationsflüssigkeit in das Rektum eingebracht.

Eine solche Anordnung, wobei der Katheterschaft in einer aus Folienlagen verschweißten, vorzugsweise beutelartig verschweißten, Folienstruktur mit mehreren ausgeformten Kompartimenten verpackt ist, welche derart um den Katheter herum angelegt sind, dass sich bei der aufeinander folgenden Anwendung der enthaltenen Elemente in der chronologischen Reihenfolge ihrer linearen Aneinanderreihung eine Abfolge von Anwendungsschritten ergibt, die mit der Benetzung der Katheterspitze mit einem Gleitmittel beginnt, sich mit der Entnahme des benetzten Katheters aus einem aufreißbaren taschenartigen Kompartiment fortsetzt und anschließend die Einbringung einer im Produkt anwendungsfertig integrierten Menge an Irrigationsflüssigkeit in das Rektum umfasst, bietet dabei eine Reihe von Vorteilen:

Durch das Einschweißen ist der Katheter hygienisch und steril verpackt.

Die enthaltenen Portionen, beispielsweise eine Gleitgels, einer Irrigationsflüssigkeit und/oder einer oder mehrerer Luft- oder Gasblasen für die Befüllung eines Ballons am Katheterschaft, können mengenmäßig genau auf den jeweiligen Anwendungsfall abgestimmt sein.

Die Abfolge der verschiedenen Schritte ist durch die geometrische Anordnung der verschiedenen Kompartimente vorgegeben, und die chronologische Reihenfolge kann beispielsweise durch einen Pfeil od. dgl. auf der beutelartigen Struktur kenntlich gemacht werden, so dass Fehlbedienungen so gut wie ausgeschlossen sind.

Beutelförmige Folienstrukturen sind leicht und benötigen wenig Platz, so dass sie überall hin mitgenommen werden können.

Indem die beiden Ballonenden sich auf das Schaftmaß des den Ballon tragenden Katheterschaftes verjüngen und in einfach umgestülpter bzw. invertierter Weise auf der vorzugsweise äußeren Mantelfläche des Katheterschaftes fixiert sind, bewegen sich bei Befüllung des Ballons die beiden radial erweiterten Ballonabschnitte in entgegengesetzten, axialen Richtungen aufeinander zu. Die beiden radial erweiterten Ballonabschnitte sind gegenüber dem verjüngten, mittigen Ballonanteil derart vergrößert, dass beim trans-anal platzierten Ballon die beiden radial erweiterten Ballonabschnitte im Laufe der Befüllung sich über den mittigen, verjüngten Ballonabschnitt stülpen und im Grenzfall einander unmittelbar berühren und dadurch ihre Relativbewegung begrenzen und das distale Ende des Katheterschaftes daran hindern, den Scheitelpunkt des intra-rektalen Ballonradius bei axial maximal ausgelenkter Lage des Schaftkörpers nach distal hin zu überschreiten.

Um schaftspitzenbedingten Läsionen weitestgehend vorzubeugen, beschreibt die Erfindung ein spezifisches, besonders vorteilhaftes Verhältnis der Länge des mittigen taillenartigen Ballonsegmentes zur beidseitigen Einstülpung (Inversionen) der Fixierungspunkte der Ballonschaftenden auf dem Katheterschaft. Dieses Verhältnis gewährleistet, dass die Spitze des Katheterschaftes sich im befüllten, trans-anal positionierten Zustand spontan in das intra-rektale Ballonsegment retrahiert und dort atraumatisch eingebettet ist. Die atraumatische Sicherung der Katheterspitze innerhalb des intra-rektalen Ballonsegmentes ist erfindungsgemäß auch dann noch gewährleistet, wenn es zu anwendungstypischen Auslenkungen der Schaftachse innerhalb des Analkanals kommt, wie sie sich leicht durch Zug oder Stoß an der katheterzuleitenden Schlauchverbindung einstellen können.

Die Erfindung geht ferner auf das Problem der Vermeidung unerwünschter bzw. verfrühter Triggerwirkungen durch den befüllten, sich intra-rektal entfaltenden Ballon ein. Die Auslösung des Defäkationsreflexes kann in der Mehrzahl der Fälle durch eine Vorformung des Ballons auf Arbeitsmaß oder auch darüber hinaus (residuale Bemaßung) vermieden werden, da die zur Verankerung und Dichtung des Katheters erforderlichen Fülldrucke den im Rektum bzw. im Bauchraum herrschenden Drucken weitgehend entsprechen bzw. nur wenige Millibar darüber liegen müssen. Die atraumatische Sicherung der Katheterspitze im Bereich des intra-rektalen Ballonsegmentes ist erfindungsgemäß bereits bei derartig geringen Fülldrucken von beispielsweise 10 bis 25 mbar gewährleistet.

Die Ausformung des Ballons auf sein Arbeitsmaß erlaubt es dem Anwender ferner, durch die Erhöhung des Fülldrucks bzw. Füllvolumens, vom initialen Füllzustand primär dichtend und ankernd wirkenden Ballons, eine gut kontrollierbare, graduell steigerbare Dehnung der dem Katheterballon anliegenden Darmwandung herbeizuführen, die schließlich, bei individuell angepasster Intensität, zur kontrollierten Auslösung des Defäkationsreflexes führt.

Neben der initialen Dehnung der Wandung des Rektums kommt es bei entsprechender weiterer Steigerung des Fülldrucks im hantelförmig taillierten Katheterballon zudem zu einer Dehnung des Anus bzw. des analen Schließmuskels, was einen weiteren effizienten Triggerstimulus darstellt.

Die Intensität einer derartigen pneumatischen Dehnung der Darmwandung bzw. des Anus kann die Intensität einer reflex-auslösenden Dehnung mit flüssigen Medien deutlich überschreiten. Auf Grund des intensiveren Stimulus kann es trotz einer nur einmalig erfolgten Stimulation zur Auslösung von mehreren sequentiell ablaufenden Entleerungsreflexzyklen kommen.

Durch den vom Anwender gut steuerbaren pneumatischen Dehnungsstimulus kann zudem in vielen Fällen die Menge der erforderlichen Einlaufflüssigkeit reduziert werden, was wiederum die für die Entleerung des Einlaufs aus dem Rektum erforderliche Zeit erheblich verkürzen kann, und das Verfahren für viele Anwender so schließlich als Therapieoption für die Selbstanwendung erschließt.

Auf eine aufwendige technische Ausführung von Irrigationsgeräten kann bei der hier dargestellten optionalen pneumatisch-flüssig kombinierten Reflextriggerung verzichtet werden, da im bevorzugten Anwendungsfall das erforderliche Irrigationsvolumen so klein gehalten werden kann, dass es als kompakt anwendungsfertig zubereitete Lösung direkt mit einem erfindungsgemäß ausgeführten Einlaufkatheter über eine feste Zuleitung verbunden sein kann, und so in idealer Weise als Einmalprodukt zur Anwendung kommen kann.

Die irrigierende Lösung ist hierzu vorzugsweise in einem zylindrischen, beutelartigen Behälter abgefüllt, der vom Anwender bequem durch Auspressen mit der Hand intra-rektal eingebracht werden kann.

Der erfindungsgemäße Einlaufkatheter verfügt desweiteren über eine bevorzugt fest eingeklebte Füllleitung zur Beaufschlagung des Katheterballons mit Fülldruck. Die Einheit zur Befüllung des Ballons ist bevorzugt in wiederverwendbarer Form ausgeführt, und kann als einfacher, über eine Kupplung anschließbarer Handpumpballon oder auch Pumpballon mit druckanzeigendem Manometer gestaltet sein. Alternativ ist auch eine volumen-kontrollierte Befüllung des Ballons denkbar. Unabhängig von der Art der Befüllung, kann der Anwender durch schrittweise Näherung, die für sich optimale Ballonfüllung ermitteln, welche seiner individuellen Anatomie und seinem jeweiligen Reflexstatus bestmöglich angepasst ist.

Zur Gewährleistung einer möglichst praktikablen Kathetereinführung sowie einer sicheren trans-analen Platzierung des verjüngten Teils des hantel-förmigen Ballonelementes ist der Katheterschaft im proximalen, prä-analen Bereich vorzugsweise mit Griffmulden zur Aufnahme der den Katheter beim Einführen greifenden Finger ausgestattet. Falls der Anwender über keine Sensibilität im Becken- bzw. Analbereich verfügt, führt er den Katheter mit den Fingern bis zum Anschlag an den Anus ein und kann so ein unkontrolliert tiefes Einführen vermeiden.

Weitere Merkmale, Eigenschaften, Vorteile und Wirkungen der Erfindung ergeben sich aus der folgenden Beschreibung bevorzugter Ausführungsformen der Erfindung sowie anhand der Zeichnung. Hierbei zeigt:
- Fig. 1a: eine Ausführungsform eines erfindungsgemäßen Katheters in einem schematischen Längsschnitt, wobei die Ballonenden in erfindungsgemäß invertierter Weise auf dem Katheterschaft aufgebracht sind;
- Fig. 1b: die geometrische Konstruktion des Scheitelpunktes des distalen Ballonradius;
- Fig.1c: den in Fig.1a beschriebenen Ballonkörper in frei entfaltetem, mit geringem Fülldruck beaufschlagten Zustand, außerhalb des Anus;
- Fig.1d: den in Fig.1a beschriebenen Ballonkörper in mit Fülldruck beaufschlagtem, trans-anal platzierten Zustand;
- Fig. 1e: die geometrische Konstruktion der distalen Tangentialebene an den intrarektalen Ballonabschnitt;
- Fig. 2: eine der Fig. 1 a entsprechende Darstellung einer anderen Ausführungsform der Erfindung, wobei eine Katheterspitze über die vordere Fixierungslinie des Ballonschaftendes hinaus reicht;
- Fig. 3: eine der Fig. 2 entsprechende Darstellung, wobei weitere Hilfslinien eingezeichnet sind, derart, dass der Bezugspunkt für die Bestimmung der Inversionstiefe B nicht der vordere Ballonradius ist, sondern der größte Durchmesser D des intra-rektalen Ballonsegmentes;
- Fig. 4: eine ausgeformte Ballonhülle in nicht befülltem Zustand, die in besonders vorteilhafter Weise für das rektale Einführen und die sichere trans-anale Platzierung und Entfaltung des Ballons vorgesehen ist;
- Fig. 5: eine abgewandelte Ausführungsform der Erfindung mit in ihrem transanalen Abschnitt tailliertem Katheterschaft;
- Fig.6: ein manuell bedienbares Pumpmanometer mit einer für eine mehrstufige, sequentielle Befüllung des Katheterballons ausgestatteten Druckskala;
- Fig. 7: eine wiederum abgewandelte Ausführungsform der Erfindung mit fest mit dem Katheterschaft verbundenem Einlaufbehälter und Füllschlauch zur Beaufschlagung des Katheterballons mit Fülldruck;
- Fig.8: zeigt eine beispielhafte Ausführungsform der Erfindung, bei der verschiedene Elemente zur Verabreichung eines trans-analen Einlaufs in einem folienverschweißten Behältnis kompartimentiert verpackt bzw. anwendungsfertig eingebracht sind;
- Fig.8a: stellt schematisch dar, wie die Folienlagen durch vorzugsweise Schweißnähte miteinander fest verbunden sind und zwischen sich ein Kompartiment ausformen; und
- Fig.9: zeigt eine weitere besondere Ausführungsform der Erfindung in weitgehender Analogie zu Abb. 8, bei der die verpackenden Anteile der Sektion 48 bereits von den die Medien beinhaltenden Kompartimenten abgetrennt sind, und die neben einem Flüssigkeit aufnehmenden Kompartiment auch mit einem oder mehreren Kompartimenten zur Aufnahme von beispielsweise Luft für die Befüllung des Katheterballons ausgestattet sind.

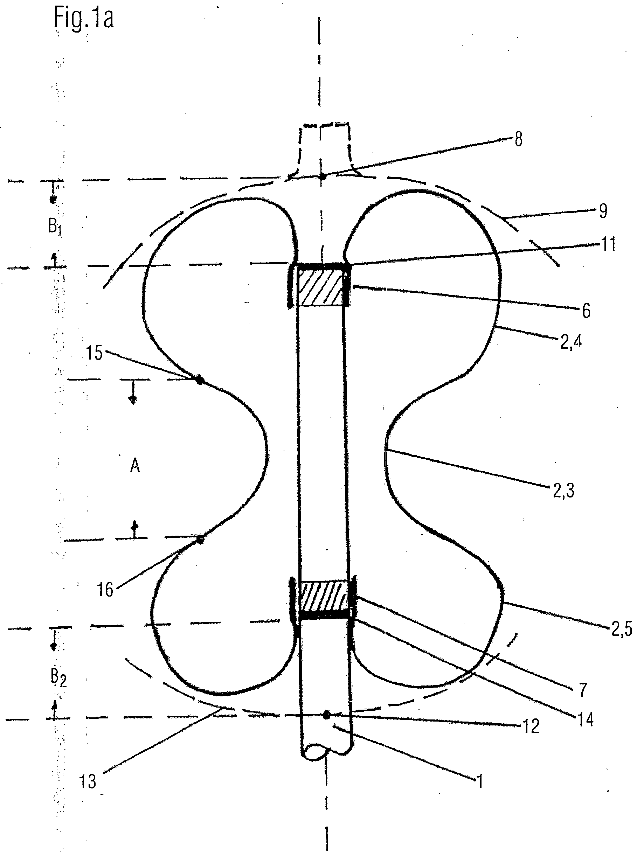

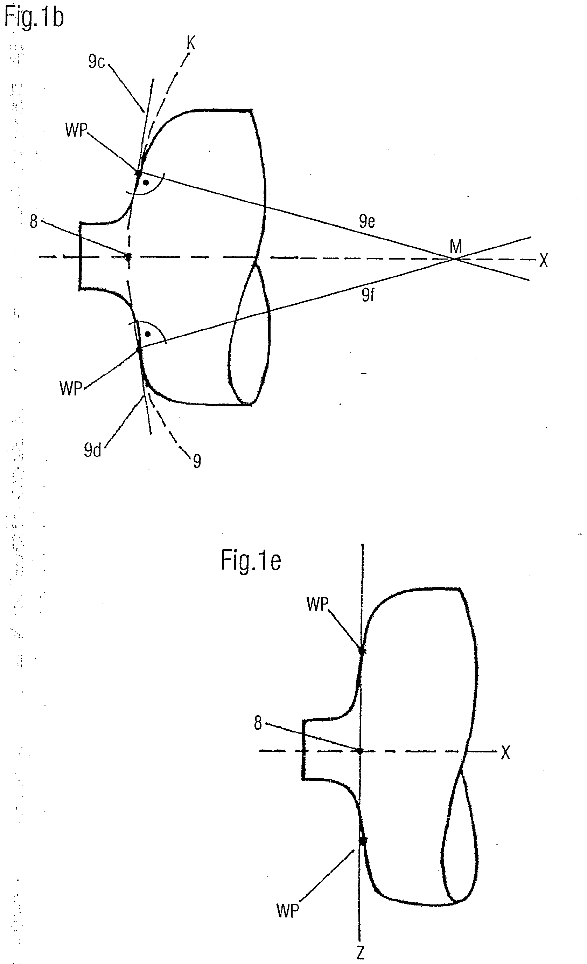

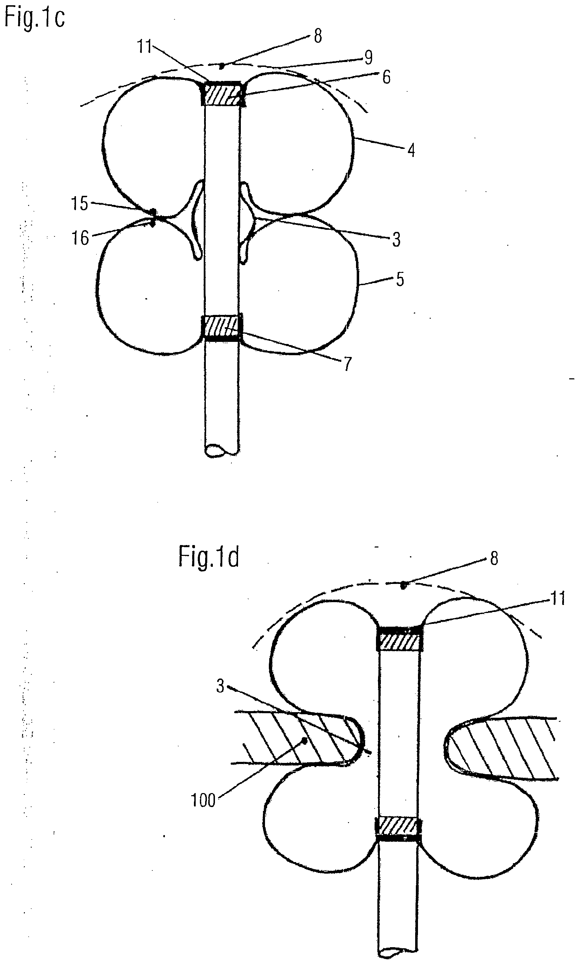

Fig. 1 a zeigt einen Längsschnitt durch einen Einlaufkatheter mit einem distal auf dem Katheterschaft 1 endständig aufgebrachten hantel- oder sanduhrförmigen Ballonelement 2. Das Ballonelement weist jeweils an den Enden beispielsweise sphärische oder diskusartige Erweiterungen auf. Im mittigen Bereich zwischen den endständigen Erweiterungen befindet sich ein im Durchmesser reduziertes, zylindrisch oder annähernd zylindrisch ausgeformtes Segment 3, welches die intra-rektale Erweiterung 4 mit der prä-analen Erweiterung 5 durchgängig verbindet.

Der Ballon 2 weist endständig zwei Ballon-Schaftenden (6,7) zur Befestigung des Ballons auf dem Schaft auf. Die Schaftenden 6 und 7 werden bei der Ballonmontage beidseitig um einen bestimmten Betrag B in das Balloninnere eingestülpt (invertiert) und in dieser, zu einander hin versetzten Position, auf dem Schaft 1 durch beispielsweise Klebung oder Verschweißung fixiert.

Die Summe der Beträge B der beidseitigen Inversion soll dabei mindestens der Länge des verjüngten Zwischenstückes A entsprechen (A kleiner/gleich Summe der Beträge B).

Die Inversionstiefe B auf der dem Patienten bzw. dem Rektum zugewandten Ballonseite, entspricht der Distanz des Scheitelpunktes 8 des distalen, intra-rektalen Ballonradius 9 zur distalen Fixierungslinie 11 des Ballonendes 6 auf dem Katheterschaft.

Der distale Radius 9 entspricht dem frontalen Radius bei freier, nicht invertierter Entfaltung des vollständig befüllten, jedoch nicht mit Druck beaufschlagten Ballons (gestrichelte Linie). Eine beispielhafte Vorschrift zur geometrischen Ermittlung des Scheitelpunktes 8 mit guter Näherung, ist in Fig. 1b dargestellt. Man erkennt insbesondere die beiden Wendepunkte WP des Längsschnitt durch das distale Ballonende, woraus durch Inversion des distalen Ballonbereichs der sphärische oder diskoide oder etwa halb-toroidale Bereich des intra-rektalen Ballonabschnitts resultiert. Die Lotrechte 9e, 9f an die Tangente an den Ballon-Längsschnitt schneiden die Längsachse X des Kathetersschaftes in dem Punkt M, und ein Kreis K um diesen Punkt M mit dem Abstand M-WP liefert den vorderen Scheitelpunkt 8, der die distale Grenze für den Katheterschaft in dessen neutraler, nicht ausgelenkter Ausgangsposition markiert.

In Fig. 1 e ist zu sehen, dass man durch die Punkte WP auch eine Gerade legen kann, welche die Ebene Z repräsentiert, die nach Umstülpung des vorderen Ballonendes gerade eben den intra-rektalen Ballonabschnitt von distal tangiet und ebenfalls als Maß für die distalste Position des Katheterschaftes in dessen neutraler, nicht ausgelenkter Ausgangsposition angesehen werden kann.

Auf der dem Patienten abgewandten Ballonseite entspricht die Inversionstiefe B der Distanz des Scheitelpunktes 12 des proximalen, prä-analen Ballonradius 13 zur proximalen Fixierungslinie14 des Ballonendes 7 auf dem Katheterschaft.

Der Radius 13 entspricht dem proximalen Radius bei freier, nicht invertierter Entfaltung des Ballons. Die geometrische Ermittlung des Scheitelpunktes 12 entspricht der in Fig. 1 b beschriebenen Näherung.

Die Bestimmung der Länge des Zwischenstückes A erfolgt durch die Bestimmung der Distanz zwischen den Übergängen der Schulterradien 15 und 16 (Wendepunkte) der einander zugewandten Schulterflächen der Ballonsegmente 4 und 5.

Die Bestimmung der Inversionstiefen, Längen und Distanzen erfolgt jeweils im gefüllten, mit Fehldruck beaufschlagten Zustand, wobei der Fülldruck so gewählt wird, dass sich der Ballon vollständig entfaltet, sich jedoch keine elastische Aufdehnung der Ballonhülle einstellt.

Die Inversionstiefe B berechnet sich wie folgt: B >= A/2 (>= entspricht: größer/gleich).

Bei der Montage des Ballons auf dem Katheterschaft ist der Bezugspunkt für die jeweilige Inversion der Ballonschaftenden zum einen der Scheitelpunkt 8 des distalen, intra-rektalen Ballonradius 9, zum anderen der Scheitelpunkt12 des proximalen, prä-analen Ballonradius 13.

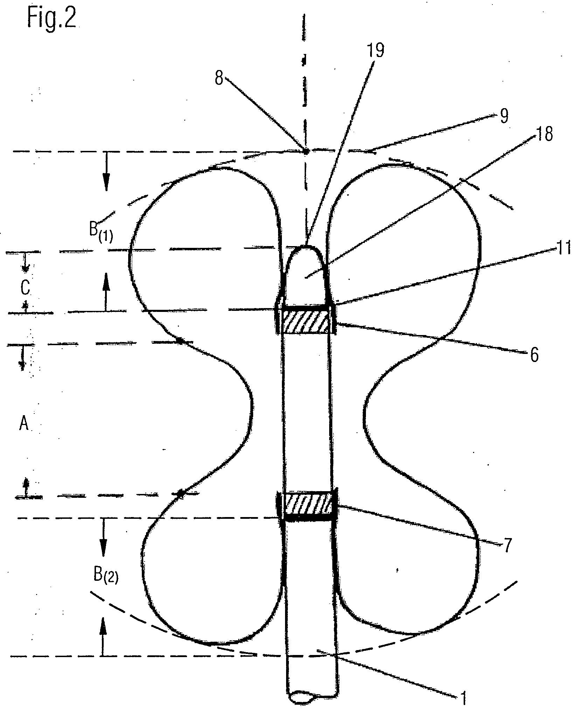

Bei der in dieser Abbildung beschriebenen Ausführungsform entspricht die distale Fixierungsline 11 auch dem distalen Ende des Katheterschaftes 1. Der Schaft schließt direkt mit der Fixierungslinie 11 ab und reicht nicht, wie in Abbildung 2 dargestellt, über diese Fixierungslinie nach distal hinaus.

Fig. 1 b beschreibt die geometrische Herleitung des Scheitelpunktes des distalen Ballonradius.

Der vordere, frontal zum Darmlumen hin weisende Ballonradius 9 des intra-rektalen Ballonsegmentes 4 ist als gestrichelte Linie dargestellt. Er wird aus den beiden Wendepunkten 9a und 9b sowie den beiden, diesen Punkten jeweils zugehörigen Wendetangenten 9c und 9d konstruiert.

Mit Hilfe einer der beiden Wendepunkte 9a oder 9b wird eine Gerade 9e oder 9f konstruiert, welche zum einen lotrecht zur jeweiligen Wendetangente 9c oder 9d ist und den entsprechenden Wendepunkt 9a oder 9b schneidet. Der Schnittpunkt dieser Geraden 9e oder 9f mit der Symmetrieachse X ergibt den Mittelpunkt des Kreises K. Der Kreis K, und damit der vordere Ballonradius 9 ergibt sich aus dem Kreismittelpunt M und den Wendepunkten 9a und 9b, welche sich auf dem Kreisumfang befinden. Der Scheitelpunkt 8 ergibt sich bei dieser Herleitung aus dem Schnittpunkt des Kreisumfangs mit der Symmetrieachse X des Ballons.

Für die im Folgenden verwendete Beschreibung des frontalen Scheitelpunktes 8 wird zur vereinfachten Herleitung des jeweils am weitesten nach distal reichenden Punktes der befüllten, nicht mit Druck beaufschlagten Ballonhülle der Schnittpunkt der Verbindungslinie Z der beiden Wendepunkte 9a und 9b mit der Symmetrieachse X verwendet.

Fig. 1 c zeigt, wie sich der in Fig. 1 a erfindungsgemäß invertierte Katheterballon bei freier Entfaltung und ohne Beaufschlagung mit Druck, im freien, nicht trans-anal platzierten Zustand verhält. Gezeigt wird die durch die spezifische Inversion der Ballonenden 6 und 7 auf dem Katheterschaft ermöglichte Gegenrollbewegung der beiden endständigen Ballonsegmente 4 und 5.

Bei einer bevorzugt besonders dünnwandigen und weichfolienartigen Ausführung des Ballonkörpers bewegen sich die beiden Segmente bereits bei geringstem, nahezu ambientem Fülldruck aufeinander zu und überrollen das mittige Segment 3. Bei Kontakt der beiden Segmente im Bereich der Übergangspunkte (15,16) der Schulterradien schließt der Scheitelpunkt 8 des Radius 9 bündig bzw. nahezu bündig mit der distalen Fixierungslinie 11 des distalen Ballonschaftendes 6 auf dem Katheterschaft ab.

Eine derartige Konfiguration würde in situ einer klinischen Anwendungssituation bei maximal verkürzter Länge des Analkanals entsprechen. Auch in diesem Extremfall wäre somit, bedingt durch die beschriebene Inversionsvorschrift gewährleistet, dass das freie distale Katheterschaftende, welches hier der distalen Fixierungslinie 11 entspricht, nicht in das Darmlumen hineinreicht und selbst bei maximaler seitlicher Auslenkung des Katheterschaftes im Rektum (Kippung des intra-rektalen Schaftanteils zur Darmwand) nicht mit der Wandung des Darms in Kontakt kommt und den distalen Ballonradius 9 als eine, Irritationen und Läsionen der Darmwand ausschließende, maximale Grenze überschreitet.

Fig.1d zeigt, wie sich die in Fig.1a beschriebene Invertierung der Ballonenden in Bezug zum distalen Katheterschaftende bei normaler oder nur geringfügig verkürzter Länge des Analkanals darstellt. Bei dieser, eher regulären klinischen Anwendungssituation stellt sich die vordere Fixierungslinie 11, welche hier wiederum dem distalen freien Ende des Katheterschaftes entspricht, deutlich in das Innere des intra-rektalen Ballons 4 versetzt dar. Die beiden endständigen Ballonsegmente 4 und 5 bewegen sich bei Beaufschlagung des Ballons mit Druck gegenläufig auf den Anus zu und schmiegen sich so dem jeweiligen analen Situs dichtend an. Bei entsprechend dünnwandiger Ausführung und weichfolienartigem Charakter der Ballonhülle stellt sich das Gegenrollen der Ballonsegmente ebenfalls bereits bei einer sehr geringen, zur Umgebung nahezu ambienten wirkenden Druckkraft (Fülldruck) ein.
Bereits der am trans-anal platzierten Ballon anliegende bzw. auf den Ballon wirkende individuelle, intra-abdominelle Druck führt zu einer kombinierten trans-analen Dichtwirkung aus radialer Dichtung gegen den Analkanal und einer von axial her gerichteten dichtenden Gegenrollbewegung der endständigen Ballonerweiterungen an den inneren und äußeren Ausgang des Anus. Die Dichtwirkung, ist daher nicht von einer initialen Befüllung des Ballons über sein Volumen bei freier Entfaltung hinaus, bis zur beginnenden Dehnung seiner Ballonhülle abhängig. Der Ballon kann sich auch bei partieller Befüllung, von beispielsweise 70 bis 90 % seines auf dem Katheterschaft frei entfalteten Volumens, in beschriebener axial gegenrollender und sich radial entfaltender Weise verhalten. Hierdurch ist eine nahezu druckneutrale und irritationsfreie Platzierung des Ballonkatheters im Anus möglich.

Eine traumatisierende Einwirkung der Katheterschaftspitze auf die Darmwandung kann somit bei normaler oder wenig veränderter analer Anatomie durch die sich erfindungsgemäß einstellende Inversion der Schaftspitze auch bei völlig druckneutraler Beaufschlagung mit einem Füllmedium ausgeschlossen werden.

Fig. 2 zeigt beispielhaft, wie Abschnitte des Katheterschaftes, welche die distale Fixierungslinie 11 in distaler, darmwärts gerichteter Fortführung als Spitzenstück 18 überschreiten, bei der Bestimmung der Inversionstiefe der Ballonenden 6 und 7 zu berücksichtigen sind, um eine erfindungsgemäßes atraumatische Inversion des distalen Katheterendes im intra-rektalen Ballon bei trans-analer Platzierung des befüllten Katheterballons zu gewährleisten. Die Länge C des Spitzenstückes 18 wird definiert als Distanz der vorderen Fixierungslinie 11 zum vorderen Scheitelpunkt 19 des Spitzenstückes.

Die Länge B verlängert sich im Vergleich zu Abb.1 a um den Betrag der Länge C bzw. C/2.

Die korrespondierende Inversionstiefe B berechnet sich bei Berücksichtigung eines Spitzenstücks bevorzugt nach: B >= A/2 + C.

Alternativ hierzu kann die korrespondierende Inversionstiefe B bei Berücksichtigung eines Spitzenstücks weniger bevorzugt nach: B >= A/2 + C/2 erfolgen.

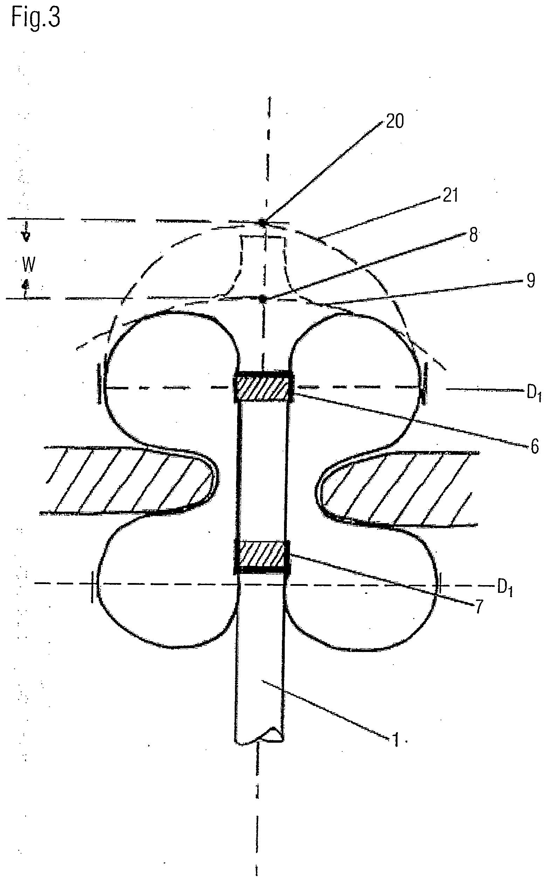

Fig. 3 stellt eine weitere, alternative Vorschrift zur Festlegung der Inversionstiefe B der Ballonschaftenden 6 und 7 auf dem Katheterschaft 1 in Bezug zum distalen Katheterschaftende 11, 19 dar.

Hierbei wird insbesondere eine mögliche axial gerichtete Verschiebung des Katheterschaftes innerhalb des befüllten, trans-anal platzierten Ballons berücksichtigt. Eine derartige Auslenkung des Schaftes in der Längsachse könnte im Rahmen der erfindungsgemäß beschriebenen Inversion der Ballonschaftenden auf dem den Ballon tragenden Katheterschaft zu darmwärts gerichteten Verschiebungen des distalen Katheterschaftende führen, und somit ein potentielles Perforationsrisiko darstellen.

Als maximaler distaler Auslenkungsweg W der vorderen Fixierungslinie 11 wird eine Distanz definiert, die sich als distal gerichtete Verlängerung der Schaftlängsachse vom Scheitelpunkt 8 des Radius 9 ausgeht und bis zum Scheitelpunkt 20 eines Radius 21 reicht, wobei der Radius 21 über dem größten Durchmesser D des intra-rektalen Ballonsegmentes 4 errichtet wird.

Verfügt der Katheterschaft über ein Spitzenstück 18, welches über die Linie 11 hinausreicht, soll der maximale Auslenkungsweg W entsprechend so gewählt werden, dass die Spitze 19 des Spitzenstücks bei maximaler Auslenkung W des Schaftes nicht über den Radius 21 reicht.

Der durch den größten Durchmesser D im intra-rektalen Ballonsegment definierte Radius 21 stellt eine prinzipiell relevante Begrenzungslinie für distale Katheterschaftanteile dar. Bei einer seitlichen Verkippung der Schaftlängsachse des trans-anal platzierten Katheterschaftes, ist durch den Bezug der maximalen Auslenkung W auf den größten Ballondurchmesser D gewährleistet, dass die Katheterschaftspitze (11,19) sich noch innerhalb des Schwenkradius 21 des Ballonsegmentes 4 bewegt, und somit ein potentiell traumatisierende Kontakt der Spitze mit der dem Ballon anliegenden Darmwand relativ gut ausgeschlossen werden kann.

Vorzugsweise soll bei der Bestimmung der Inversionstiefe B das jeweilige Verhältnis der Distanz W zum Radius 21 bzw. des ihm zugrundeliegenden Durchmessers D eingehalten und die Inversionstiefe B bei Bedarf entsprechend angepasst werden.

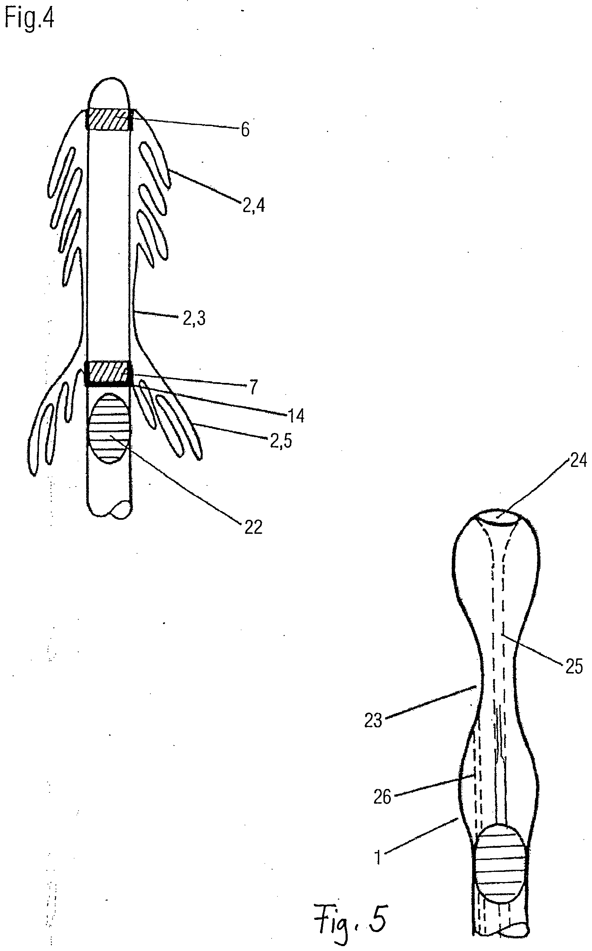

Fig. 4 zeigt den Katheterballon 2 in entleerter, dem Katheterschaft anliegender Form, wie er für die Einführung in den Anus bereitgestellt wird. Die Hüllensegmente des intra-rektalen Ballons 4 sowie des mittigen Segmentes 3 liegen dem Schaft in Faltung angeschmiegt an.

Dabei kommen die beiden Hüllenanteile vorzugsweise etwa auf Höhe der Strecke zwischen den Fixierungspunkten der Ballonenden 6 und 7 auf der Schaftoberfläche zu Liegen. Die Hülle des prä-analen Ballonsegmentes 5 wird hingegen bevorzugt nach proximal ausgestrichen und überragt die den Katheter beim Einführen greifenden Finger, wobei der bevorzugte Griffpunkt sich unmittelbar proximal von der proximalen Fixierungslinie 14 befindet. Der Griffpunkt 22 ist bevorzugt als muldenartige Aufnahmefläche ausgeführt, welche bevorzugt auf den beiden, um 180 Grad versetzten, gegenüberliegenden Schaftflächen aufgebracht sind.

Der Anwender greift bei einer derartigen Fixierung des evakuierten Ballons mit seinen Fingern unter die nach proximal ausgestrichene Hülle des Segmentes 5 und führt den Katheter bis zum Anschlag der greifenden Finger am äußeren Anus in das Rektum ein. Somit ist eine definierte Einführtiefe gewährleistet. Ferner ist sichergestellt, dass der intra-rektale Ballonanteil 4 in die rektale Kavität eingeführt ist, während der proximale Ballonanteil 5 außerhalb des Anus (prä-anal) zu liegen kommt. Bei der Befüllung des taillierten Ballons stellt sich somit eine sichere transanale Positionierung des Katheters ein.

Fig. 5 zeigt eine bevorzugte Ausführung des Schaftkörpers 1, die bereits eine gewisse selbsttätige Positionierung und Sicherung des Schaftes im Anus bietet, wenn der Katheterballon noch nicht befüllt ist. Der Schaft 1 weist hierzu eine ebenfalls taillierte Form auf, die im trans-analen Bereich 23 entsprechend verjüngt ist, und den Katheterschaft in diesem Bereich nach dem Einführen quasi in trans-analer Position einrastet. Der Katheterschaft weist bevorzugt zudem eine distal endständige, trichterartig mündende, atraumatisch ausgeformte Öffnung 24 auf, der sich der das Medium zuleitende Kanal 25 anschließt.

Bei einer besonders großvolumigen Ausführung des intra-rektalen Ballonsegmentes bzw. einer weit in das Rektum reichenden Längsausdehnung des Ballonsegmentes, kann dieses im entlüfteten, anwendungsfertigen Zustand optional partiell in die Öffnung 24 hineingestopft bzw. verpackt sein. Bei Befüllung des eingeführten Katheters schlüpft sie dann aus der Öffnung heraus.

Die Taille 23 kann zudem, bei angepasster Ausführung des Schaftmaterials eine gewisse Knickbarkeit des Schaftkörper vorgeben, was dessen atraumatische Eigenschaften verbessert.

Die Befüllung des Ballons erfolgt durch einen separaten schaftintegrierten Kanal 26.

Zur Vermeidung des Rückstroms von Irrigationsflüssigkeit kann der Einlaufkatheter im Bereich des flüssigkeitsführenden Kanals 25 mit einem Rückschlagventil ausgestattet ist.

Das Ventil kann vorzugsweise aus einem dünnwandigen Schlauchelement von wenigen, vorzugsweise 5 bis 15 Mikrometern Wandstärke und dem Durchmesser des Kanals 25 bestehen, wobei das distale Ende des Schlauches über eine Länge von ca. 5 bis 10 mm frei im Kanal 25 liegt, und dessen proximales Ende dicht schließend mir der Innenwandung des Kanals 25 verbunden ist. Bei spitzenwärts gerichtetem Durchfluss des Mediums durch den Kanal öffnet sich das Schlauchelement und erlaubt den freien Durchfluss des Mediums. Bei entgegengesetzt gerichtetem Fluss kollabiert das Schlauchelement und verschließt sich dichtend, einen effektiven Rückstrom verhindernd.

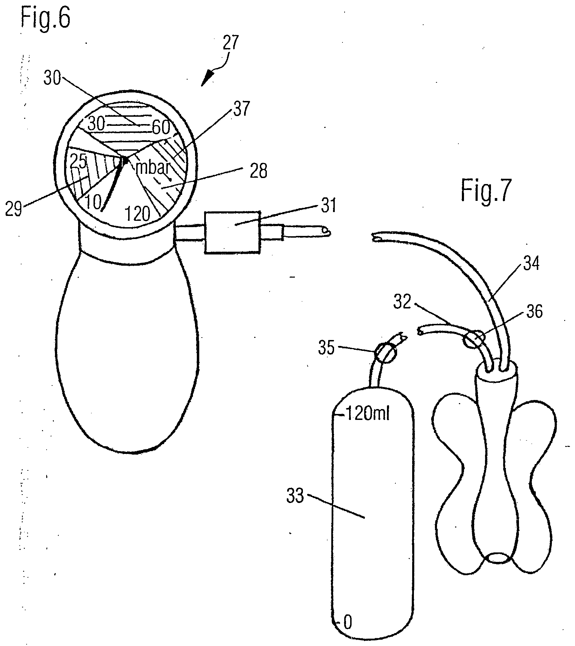

Fig. 6 zeigt schematisch ein Pumpmanometer 27 mit einer, zu einer mehrschrittigen bzw. sequentiell den Fülldruck steigernden Befüllung des erfindungsgemäßen Katheterballons geeigneten Skala 28. Die Skala stellt bevorzugt zum einen, einen initialen, niedrigen Druckbereich 29 (ca. 10-25 mbar) dar, der vom Anwender nach dem Einführen des Katheters und vor dem Einbringen der Einlaufflüssigkeit eingestellt wird, und bei erfindungsgemäßer Vorformung und Schaftfixierung der Ballonhülle in der Mehrzahl der Fälle bereits eine trans-anale Ankerung und Dichtung des Katheters gewährleistet, ohne einen unmittelbaren reflex-triggernden Effekt auf die Darmwand zu haben.

Bei einer durch den Anwender in Folge ausgelösten Steigerung des Fülldrucks im Ballon in den Bereich 30 (30-60 mbar, mit anzunehmender korrespondierender Dehnung rektaler Darmwandanteile) oder in den Bereich 37 (60-120 mbar, mit anzunehmender zusätzlicher Dehnung des analen Sphinkters) kann der Anwender schließlich einen in seiner Intensität weitgehend reproduzierbaren Triggerstimulus zur Auslösung eines Defäkationsreflexes erzeugen. Der Anwender hat somit den Vorzug, bei einem initial niedrigen, lediglich ankernd und dichtend wirkenden Ballondruck einen Entleerungsreflex weitgehend vermeiden zu können, und damit die Einlaufflüssigkeit für seinen individuellen Bedarf auseichend lange im Darm halten zu können, was zu einer besseren Lösung bzw. Suspension des Stuhls in der Flüssigkeit führt. Andererseits kann er durch eine willkürlich herbeigeführte Druckerhöhung im Ballon einen intensiven, relativ prompt wirkenden, reflex-auslösenden Stimulus erzeugen, der hinsichtlich seiner Intensität, bei Bedarf, über die Triggerwirkung einer colo-rektalen Flüssigkeitssäule hinausgeht.

Die Befüllung des Katheterballons erfolgt bevorzugt durch Luft über eine in die Schaftwandung des Katheterkörpers 1 integrierte Füll-Leitung.

Neben einer, wie in Abbildung 6 dargestellten druckkontrollierten Befüllung des Ballons mit einem Pumpmanometer, kann zur volumen-kontrollierten Befüllung ein beigefügtes Spritzenelement verwendet werden, welches das bevorzugte Füllvolumen durch eine entsprechende Markierung auf dem Spritzenkörper vorgibt. Die Befüllung des Ballons erfolgt bei einer ein-schrittigen Befüllung bevorzugt partiell. Der Ballon liegt im idealen Fall also den Strukturen des Darms und des Anus in schlaffer, nicht-gedehnter Form an. Die Ballonhülle nimmt so die jeweilig im Rektum, im Anus und von prä-anal auf den Ballon einwirkenden Kräfte auf, und bringt den Ballon in seine, die ankernde und dichtende Funktion ausübende Konfiguration. Die jeweiligen physiologisch wirkenden Kräfte werden vom Katheterballon aufgenommen und so eine weitestgehend druckneutrale ano-rektale Ballonplatzierung ermöglicht, was die Wahrscheinlichkeit ungewollter und verfrühter Triggerwirkungen weitgehend ausschließt.

Eine volumenkontrollierte Füllung des Ballons kann ebenfalls zwei-schrittig erfolgen, wobei initial eine inkomplette Befüllung erfolgt, und der Ballon im zweiten Füllschritt mit einem triggernd wirkenden Volumen befüllt wird. Die sich bei den jeweiligen Volumina im trans-analen Ballon einstellenden Drucke sollen dabei bevorzugt in die in Fig. 6 beschriebenen Druckbereiche (29,30) fallen, und sind vom Anwender, wie bei der druckkontrollierten Befüllung, für sich individuell zu ermitteln.

Zur Begrenzung des Fülldrucks bzw. Vermeidung kritisch hoher Ballonfülldrucke kann sowohl bei manometer- als auch spritzenbetätigter Füllung ein Druckbegrenzungsventil 31 zwischen Füllelement und Katheter eingefügt werden, welches beispielsweise Ballonfülldrucke über 120 mbar vermeidet.

Fig. 7 zeigt eine bevorzugte Ausführungsform eines Einlaufkatheters, der am proximalen Ende in zwei, vorzugsweise fest mit dem Schaft verbundene Zuleitungen übergeht, wobei die Zuleitung 32 mit einem vorzugsweise beutelartigen Behälter 33 mit Einlaufmedium fest verbunden ist, und somit eine für die Anwendung gebrauchsfertige Einheit von Katheter und Medium darstellt.

Das Volumen des Behälters 33 ist dabei so bemessen, dass dieser ca. 80 bis 120 ml Irrigationslösung aufnimmt. Die relativ kleine Menge wird vom Anwender manuell ausgepresst und so, durch wiederholtes Pressen in das Rektum eingebracht. Um ein Greifen des Behälters auch bei eingeschränkter Handmotorik zu ermöglichen, ist der Behälter vorzugsweise zylindrisch, mit einem Durchmesser von ca. 4-6 cm aus geformt.

Die Verbindung 32 ist vorzugsweise mit einem durch Knickung zerbrechbaren Siegel 35 ausgestattet sein, welches die Irrigationslösung frei gibt. Ebenfalls vorteilhaft ist ein im flüssigkeitsführenden Schenkel der anwendungsfertigen Vorrichtung integriertes Ruckschlagventil 36, welches die gerichtete Entleerung des Behälters ohne Rückstrom ermöglicht.

Eine weitere Zuleitung 34 kann direkt mit einem Pumpmanometer 27 oder einer Füllspritze verbunden werden.

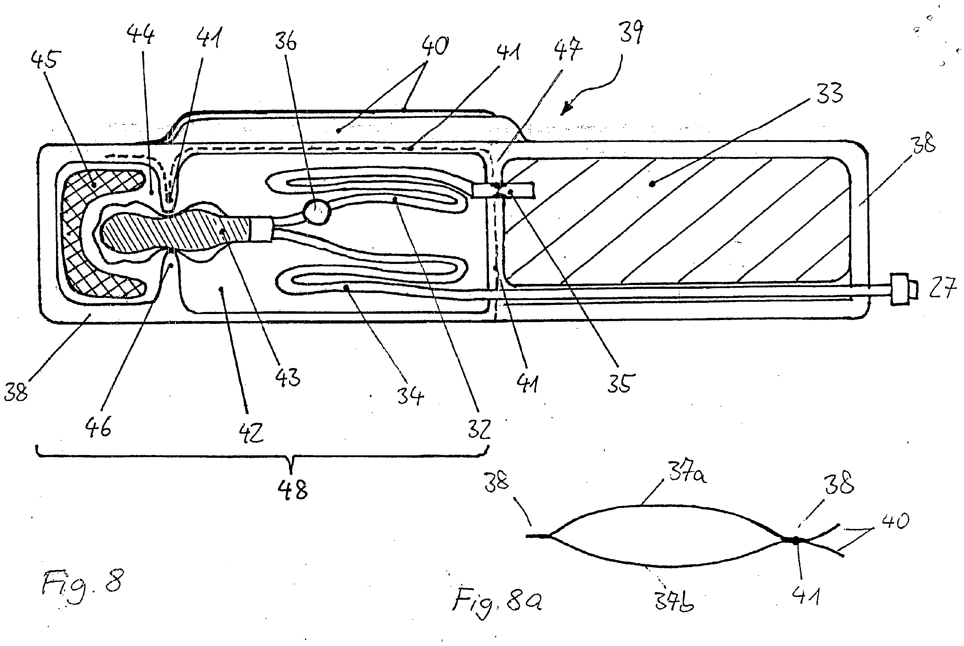

Fig.7 zeigt eine Ausführungsform der Erfindung, die in besonderer Art und Weise auf die Anwendung einer vorgefertigten Einheit von Katheter und Irrigationsvolumen eingeht, wobei die Einheit die funktionsrelevanten Elemente und Strukturen, welche über den Einlaufkatheter selbst hinaus, als Kompartimente oder mediumführende Zuleitungen darstellt, welche durch flächige, punktuelle oder lineare Verschweißung 38 beispielsweise zweier Folienlagen 37a und 37b entstehen, und in Art einer Aufreißpackung vom Patienten zur Anwendung kommen, wie dies konzeptionell in Fig. 8a dargestellt ist.

Abbildung 8 zeigt eine derartig folienverschweißte Struktur 39, deren beide Folienlagen durch Aufreißlaschen 40 und beispielsweise präformierte Schwächungen 41 der Verschweißungslinien 38 in bestimmten Bereichen vom Anwender geöffnet werden können, um so beispielsweise ein Katheterelement 43 mit daran verbundener Schlauchzuleitung (32, 34) aus einem taschenartigen Kompartiment 42 entnehmen zu können.

Um alle für den Anwender erforderlichen Komponenten bei der Anwendung einer derartigen Aufreißpackung möglichst in die Folienstruktur 39 zu integrieren, schlägt Abb. 8 beispielhaft eine spezifisch Anordnung von funktionellen Elementen vor. So ist der intra-rektal zu platzierende Anteil des Einlaufkatheters bzw. sein intra-rektales Ballonelement 4 in einem gesonderten Kompartiment 44 gelagert, in welchem es zum Beispiel durch ein angeschlossenen oder darin eingebrachtes Gelreservoir 45 unmittelbar vor der Anwendung vom Anwender durch Ausdrücken des Inhaltes mit Gleitgel benetzt werden kann. Das Kompartiment 44 und das Kompartiment 42 sind hierbei bevorzugt durch eine taillierte Abschweißung der beiden Folienlagen der Struktur 39 verbunden, die dem Schaft des Katheters möglichst eng anliegt, und ein Übertreten von Gel verhindert bzw. reduziert.
Im gegenüberliegenden Anteil der Struktur 39 kann zusätzlich ein Kompartiment 33 ausgebildet dein, das bereits die für die Anwendung optional erforderliche Irrigationsflüssigkeit enthält. Dieses Kompartiment ist mit dem Katheter durch eine entsprechende Schlauchzuleitung 32 fest verbunden. Ferner ist am proximalen Ende des Katheterschaftes ein Schlauchelement 34 angeschlossen, welches den Anschluss an eine externe Befüllvorrichtung 27 erlaubt. Die Leitung 43 ist dabei bevorzugt so angeordnet, dass sie endständig im Bereich des Kompartiments 33 aus der Folienstruktur 39 herausragt.

Die Anwendung der beschriebenen Aufreiß- und Wegwerf-Folienstruktur 39 stellt sich im Ablauf wie folgt dar. Der Anwender eröffnet das Gelreservoir 45 durch Auspressen oder Zerquetschen, und benetzt so den intra-rektalen Katheteranteil innerhalb des Kompartimentes 44. Anschließend eröffnet mit beiden Händen, die Laschen 40 greifend, das Kompartiment 42, in welchem das distale Ende des Katheters 43 griffbereit exponiert ist und die Schlauchzuleitungen 32 und 34 in platzsparend kompakter Weise verpackt sind. Die Eröffnung der Folienstruktur 39 wird präformiert über entsprechende Schwächungen 41 der Schweißlinien 38. Die Schwächungen sind dabei bevorzugt so ausgebildet, dass sie in den Bereich der Taillierung 46 bzw. darüber hinaus in den Bereich 44 reichen, und so die Entnahme des distalen Katheteranteils aus der Folienstruktur 39 erleichtern. Die Schwächungen 41 können ebenfalls in den Übergangsbereich 47 zwischen Kompartiment 42 und Kompartiment 33 fortreichen, so das im Anschluss an die Entnahme des Katheters 43 der gesamte vordere Anteil 48 der Folienstruktur 39 abgetrennt und verworfen werden kann, so dass nur noch der Katheter 43 das mit dem Katheter verbundene Kompartiment 33 verbleiben.

Der Anwender entnimmt also den Katheter bzw. legt diesen durch Abtrennen des Anteils 48 der Folienstruktur 39 frei. Er verbindet dann den Schenkel 34 zur Belüftung des Ballons mit einer endständig am Schenkel angebrachten Befüllvorrichtung 27. Er führt dann den benetzten Katheter in den Anus ein, und befüllt den Ballonanteil des Katheters mit einem Volumen, welches den Katheter im Anus verankert und gegen Darminhalt bzw. eingebrachte Irrigationslösung dichtet. Im Anschluss bricht er ein Siegel 35, welches im gebrochenen Zustand den Ausstrom von Flüssigkeit aus dem Kompartiment 33 in den Darm erlaubt. Der flüssigkeitsführende Schenkel 32 ist ferner bevorzugt mit einem Rückschlagventil 36 oder einem andersartig den Rückstrom von Flüssigkeit verhindernden Element ausgestattet. Im Anschluss an das Auspressen der Flüssigkeit in den Darm und einer gewissen Einwirkzeit im Darm, kann der Anwender durch Erhöhung des Füllvolumens im Katheterballon eine reflextriggernde Dehnung der ballonexponierten Darmanteile herbeiführen. Setzt diese ein, wird der Ballon zur Umgebung hin geöffnet, und im geöffneten Zustand aus den Anus herausgezogen, wodurch sich der Darminhalt entleeren kann.

Alternativ kann die Folienstruktur 39 auch nur den Katheter 43 beinhalten, also auf ein flüssigkeitsbefülltes Kompartiment verzichten, und im Wesentlichen ausschließlich aus einem dem Abschnitt 48 entprechenden Segment bestehen.

Die Zuleitungen 32 und 34 sind im Bereich des Kompartimentes 42 vorzugsweise in gewickelter oder gefalteter Form platzsparend verstaut und lassen eine Entfaltung auf ein anwendungsentsprechendes Längenmaß von ca. 50 bis 80 cm zu.

Bei der beschriebenen Ausführungsform ist eine einfache, über das Produkt hinweg gerichtete Folge von Anwendungsschritten gewährleistet, die linksseitig beim Gelreservoir 45 beginnt (Benetzung des distalen Katheterendes), sich nach rechts über das den proximalen Katheter und das zuführende Schlauchelement aufnehmende Kompartiment 42 (Entnahme des benetzten Katheters) sowie das die Irrigationsflüssigkeit aufnehmende Kompartiment 33 (Einpressen der Flüssigkeit) fortsetzt.

Fig. 8a stellt schematisch dar, wie die Folienlagen 37a und 37b der Folienstruktur 39 durch vorzugsweise Schweißnähte 38 miteinander fest verbunden sind und zwischen sich ein Kompartiment ausformen.

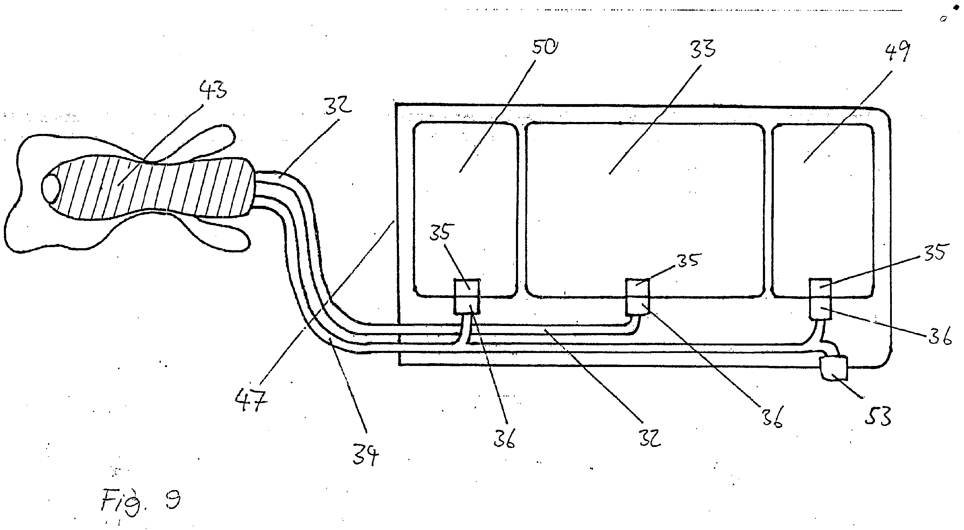

Abb. 9 beschreibt eine vergleichbare Vorrichtung wie Abb. 8, wobei hier allerdings ein oder mehrere Kompartimente zusätzlich in die Folienstruktur 39 eingebracht sind, die bereits ein Luftvolumen für die Befüllung des Katheterballons enthalten, wodurch auf den Anschluss einer externen Befüllvorrichtung gänzlich verzichtet werden kann. Die Kompartimente sind dabei bevorzugt derart angeordnet, dass sich durch ihr Auspressen in einer bestimmten linearen Abfolge, die für das Verfahren erforderliche bzw. richtige Sequenz von Anwendungsschritten einstellt. So kann beispielsweise das Kompartiment 50 ein Luftvolumen enthalten, das für die initiale Ankerung und Dichtung des Katheters im Anus erforderlich ist. Nach rechts, also vom Patienten weggerichteten Ende der Folienstruktur 39 hin, schließt sich dann das mit Flüssigkeit befüllte Kompartiment 33 an. Wiederum echts davon kann ein weiteres luftbefülltes Kompartiment 49 angeordnet sein, welches durch Auspressen das Volumen im Katheterballon weiter vergrößert, und so den Triggerreiz setzt. Die einzelnen Kompartimente sind mit Brechsiegeln anlog zu Siegel 35 zu eröffnen. Alle Kompartimente verfügen über ein den Rückstrom von Luft bzw. Flüssigkeit verhinderndes Element analog zu Element 36. Ferner ist die luftführende gemeinsame Zuleitung von den Kompartimenten 49 und 50 zum Katheter 43 mit einer Entlüftungsleitung 53 verbunden, die es ermöglicht das Füllvolumen des Ballons zur Umgebung in zu entlassen, und den Ballon so zu entleeren. Die Leitung 53 muss dabei derart mit dem Katheterballon kommunizieren, dass ein freier Ausstrom von Füllvolumen aus dem Ball on gewährleistet ist.

Hier ist, analog zu Fig. 9, ebenfalls eine anwendungsentsprechende Anordnung der Kompartimente bzw. Komponenten gegeben, wobei die von links (patientenzugewandte Seite) nach rechts angelegte Abfolge der Kompartimente bzw. Komponenten der richtigen Abfolge von Anwendungsschritten entspricht. Beginnend mit der Benetzung, gefolgt von der Katheterentnahme, der initialen Befüllung des Ballons, der Einbringung von Flüssigkeit in den Darm sowie einer optionalen zweiten triggernden Ballonbefüllung.

Neben einer seriellen Anordnung der Kompartimente ist ebenfalls eine konzentrische Anordnung denkbar, bei der beispielsweise das luftbefüllte Kompartiment 50 im Inneren eines mit Irrigationsflüssigkeit befüllten Kompartimentes 33 als von diesem separiertes Folienkompartiment angebracht ist. Auch hier wären beide Kompartimente jeweils mit einem Bruchsiegel und einem rückflussvermeidenden Element ausgestattet. Die beschriebene konzentrische Anordnung kann im Sinne der in Fig. 9 dargestellten Ausführung auch drei Kompartimente enthalten.

Die in Fig. 8 und 9 beschriebenen Ausführungsformen der kompartimentierten Folienstrukturen, die verschiedene anwendungsrelevante Komponenten in einer einzigen verpackenden bzw. bevorrätigenden Einheit zusammenfassen, können in entsprechender Weise auch bei Einlaufkatheterelementen mit einem konventionellen intra-rektalen Ballon ohne trans-analem oder prä-analem Ballonanteil bzw. auch bei Katheterelementen ganz ohne jegliche dichtende oder verankernde Ballonkomponente vorteilhaft zur Anwendung kommen.

Das Ballonelement 2 besteht aus einer dünnwandigen Weichfolie im Wandstärkenbereich von 5 bis 100 Mikrometern. Vorteilhaft sind Folien im Stärkenbereich von 5 bis 40 Mikrometern. Besonders bevorzugt sind wiederum Wandstärken von 5 bis 15 Mikrometern.

Die Verwendung von nur gering volumen-dehnbarer Materialien wie z.B. Polyurethan (PUR), beispielsweise der Spezifikation Pellethane 2363 80A bis 90A, Dow Chemical Corp., wird bevorzugt, da diese Materialien auch bei Ballonfolien im niedrigsten Wandstärkenbereich im Druckbereich von ca. 10 bis 120 mbar eine gute Formstabilität aufweisen.

Solche dünnwandigen, in komplexer Form ausgeformte PUR-Ballonfolien können bevorzugt durch Blasformung ("hot-molding") aus zuvor extrudiertem Rohschlauchmaterial hergestellt werden, was, bei entsprechender Streckung des Schlauchrohlings vor der Temperierung, eine polymere Orientierung ermöglicht, und den geformten Ballonfolien eine außerordentliche mechanische Festigkeit verleiht.

Denkbar ist ebenfalls die Verwendung von Polyurethanen niedriger Shore Härten, wie z.B. des Bereichs von 60 bis 75A, die den Katheterballon im Wandstärkenbereich von unter 40 µm, und bevorzugt unter 15µm, im anwendungstypischen Fülldruckbereich von 10 bis 120 µm, bei relativem Verlust an Formstabilität, mit einem volumendehnbaren Verhalten ausstatten.

Alternativ können beispielsweise auch nicht-volumendehnbare Materialien, wie Polyethylene, PVC oder Mischungen der genannten Materialien mit Polyurethan verwendet werden.

Erfindungsgemäße Ballonfolien können auch direkt aus der extrudierten, noch weichen, weitgehend amorphen Schlauchmasse geformt werden (in-line molding), wobei die erreichbaren Festigkeiten der Folien deutlich kleiner als bei vorextrudierten Schläuchen sind bzw. die erreichbaren Wandstärken deutlich höher als bei der Formung aus vorextrudiertem Material sind.

Denkbar für die Herstellung sind auch Tauchverfahren bei Verwendung flüssiger PVC oder PUR Materialien.

Auch das Verschweißen von singulären Folienlagen zu Ballonkörpern ist vorstellbar.

Die Verbindung des Ballons mit dem Schaftkörper erfolgt durch Verklebung, durch thermische Verfahren oder alternativ auch durch Aufschrumpfen der Ballonenden auf den Schaftkörper.

Der intra-rektale Ballonanteil 4 soll im frei entfalteten, nicht mit Druck beaufschlagten Zustand, bevorzugt einen Durchmesser von ca. 30-60 mm haben, im mittigen Taillenbereich 3 etwa 10 bis 30 mm aufweisen und im prä-analen Bereich 5 etwa 30-50 mm. Das mittige Segment 3 soll ca. 20-40 mm Länge aufweisen, die endständigen Segmente 4 und 5 jeweils eine Länge von ca. 20-40 mm.

Ist der Katheterballon in besonderer Weise zur sequentiellen Befüllung, mit einer optionalen intra-rektalen Ballontriggerung vorgesehen, hat der intra-rektale Ballonanteil 4 im frei entfalteten, nicht mit Druck beaufschlagten Zustand, bevorzugt einen Durchmesser von ca. 40-80 mm und weist eine Länge von bevorzugt 30-60 mm auf.

Neben der trans-analen Anwendung des Einlaufkatheters, können die erfindungsgemäßen Ausführungen auch für die perforationssichere Platzierung eines trans-anal eingelegten Drainagerohres unter anderem auch für die dauerhafte Ableitung von Stuhl aus dem Darm eines Patienten verwendet werden. Ferner ist eine Anwendung der beschriebenen Kathetertechnik in chirurgisch angelegten Stomata/Öffnungen oder weiteren natürlichen Körperöffnungen denkbar.

Die erfindungsgemäße Folienstruktur 39 umfasst mindestens zwei miteinander verschweißte Folienlagen luftdichter Folien, beispielsweise sog. Hochbarrierefolien oder Aluminiumfolien. In besonderen Fällen können auch mehr als zwei Folienlagen miteinander verschweißt sein.

## Patentansprüche

1. Vorrichtung zum trans-analen Einbringen eines Einlaufs in das Rektum bzw. Colon eines Patienten, mit einem trans-anal platzierten aufblasbaren Ballon von einer hantel- oder sanduhrförmigen Gestalt, wobei die Ballonenden auf dem Körper des Katheterschaftes derart invertiert positioniert sind, dass sich die endständigen Ballonanteile bei Befüllung über den mittigen, verjüngten Ballonanteil hinweg, in gegengerichteter Weise aufeinander zu bewegen, und die distale Öffnung oder das distale Ende des Katheterschaftes dabei den Scheitelpunkt des vorderen, frontalen Ballonradius bei axial nicht-ausgelenkter Ruhelage des Schaftkörpers im in situ trans-anal platzierten Ballon nicht nach distal hin überschreitet, **gekennzeichnet durch** eine aus Folienlagen verschweißte, vorzugsweise beutelartig verschweißte, Folienstruktur (39) mit mehreren ausgeformten Kompartimenten, worin der Katheterschaft sowie ein Gleitmittel verpackt ist, wobei die Kompartimente derart um den Katheter herum angelegt sind, dass sich bei der aufeinander folgenden Anwendung der enthaltenen Elemente in der chronologischen Reihenfolge ihrer linearen Aneinanderreihung eine Abfolge von Anwendungsschritten ergibt, die mit der Benetzung der Katheterspitze mit dem Gleitmittel beginnt, sich mit der Entnahme des benetzten Katheters aus einem aufreißbaren taschenartigen Kompartiment fortsetzt und anschließend die Einbringung einer im Produkt anwendungsfertig integrierten Menge an Irrigationsflüssigkeit in das Rektum umfasst.

2. Vorrichtung nach Anspruch 1, **dadurch gekennzeichnet, dass** der vordere Teil des Katheters im Bereich von dessen Spitze sich in einem anderen Kompartiment befindet als dessen rückwärtiger Bereich.

3. Vorrichtung nach Anspruch 1 oder 2, **dadurch gekennzeichnet, dass** sich in einem die Katheterspitze aufnehmenden Kompartiment ein Gelkissen befindet mit einer benetzenden Flüssigkeit.

4. Vorrichtung nach einem der Ansprüche 1 bis 3, **dadurch gekennzeichnet, dass** eine Zwischenwand zwischen einen den vorderen Teil des Katheters aufnehmenden Kompartiment und einem dessen rückwärtigen Bereich aufnehmenden Kompartiment eine Öffnung aufweist, durch welche sich der Katheter hindurch erstreckt, wobei diese Öffnung vorzugsweise gegenüber dem Katheter abgedichtet ist.

5. Vorrichtung nach einem der vorhergehenden Ansprüche, **dadurch gekennzeichnet, dass** wenigstens ein, den Katheter oder Teile desselben ursprünglich aufnehmendes Kompartiment entlang einer Aufreißlinie, bspw. in Form einer Perforierungslinie, aufreißbar ist, um den Katheter entnehmen zu können.

6. Vorrichtung nach einem der vorhergehenden Ansprüche, **dadurch gekennzeichnet, dass** ein oder mehrere, den Katheter ursprünglich aufnehmende Kompartimente mittels Sollbruchstellen, bspw.in Form von Perforierungslinien od. dgl., mit weiteren Abschnitten oder Kompartimenten der Folienstruktur (39) verbunden sind, so dass die Hülle der den Katheter ursprünglich aufnehmenden Kompartimente von den übrigen Abschnitten oder Kompartimenten der Folienstruktur (39) trennbar, insbesondere abreißbar sind.

7. Vorrichtung nach einem der vorhergehenden Ansprüche, **dadurch gekennzeichnet, dass** sich ein oder mehrere Verbindungsschläuche in einem gemeinsamen Kompartiment mit dem Katheter, insbesondere mit dem rückwärtigen Bereich des Katheters, befinden.

8. Vorrichtung nach einem der vorhergehenden Ansprüche, **dadurch gekennzeichnet, dass** ein Verbindungsschlauch zu einem Flüssigkeitsreservoir führt, insbesondere zu einem Flüssigkeitsreservoir in einem dritten Kompartiment.

9. Vorrichtung nach einem der vorhergehenden Ansprüche, **dadurch gekennzeichnet, dass** das Flüssigkeitsreservoir derart bemessen ist, dass das darin enthaltene Volumen für einen Irrigationsvorgang ausreichend ist.

10. Vorrichtung nach einem der vorhergehenden Ansprüche, **dadurch gekennzeichnet, dass** ein oder mehrere weitere, mit Luft oder einem anderen Gas gefüllte Kompartimente vorgesehen sind, womit sich ein Ballon der Vorrichtung aufblasen lässt, um diesen in einem Patienten zu verankern und/oder um den Defäkationsreflex auszulösen.

11. Vorrichtung nach einem der vorhergehenden Ansprüche, **dadurch gekennzeichnet, dass** ein oder mehrere, mit Luft oder einem anderen Gas gefüllte Kompartimente über eine Verbindungsleitung mit einem Ballon in oder an dem Katheter zu dessen Befüllung verbunden sind.

12. Vorrichtung nach einem der vorhergehenden Ansprüche, **dadurch gekennzeichnet, dass** in oder an einem Verbindungsschlauch von einem Luft-, Gas- oder Flüssigkeitsreservoir oder -kompartiment zu dem Katheter ein Rückschlagventil angeordnet ist.

13. Vorrichtung nach einem der vorhergehenden Ansprüche, **dadurch gekennzeichnet, dass** ein Verbindungsschlauch mit einem Anschluss für eine vorzugsweise externe Druckluftquelle vorgesehen ist.

14. Vorrichtung nach einem der vorhergehenden Ansprüche, **dadurch gekennzeichnet, dass** Folienlagen der Folienstruktur (39) an ihrem äußeren Rand und/oder entlang von inneren Trennlinien luftdicht verschweißt sind.

15. Vorrichtung nach einem der vorhergehenden Ansprüche, **dadurch gekennzeichnet, dass** ein oder mehrere Aufreißlinien, insbesondere Perforationslinien, auf oder entlang von Schweißlinien verlaufen, insbesondere im Bereich von inneren Trennlinien, wo die Folien luftdicht miteinander verschweißt sind.

## Claims

1. Apparatus for the transanal instillation of an enema into the rectum respectively colon of a patient by means of a transanally placed inflatable balloon of dumbbell or hourglass-shaped form, where the balloon ends are invertedly positioned on the body of the catheter shank so that the terminal balloon sections each are moving in opposite direction towards each other over the centrally located narrowed balloon section during filling, and thereby the distal opening or the distal end of the catheter shank does not protrude in distal direction beyond the vertex of the forward frontal balloon radius while the shank body is not deflected in rest position in the in situ transanally placed balloon, **characterized by** a welded, preferably bag-like welded foil structure (39) of foil layers with several fully shaped compartments, in which the catheter shank as well as the lubricant is packed, in which the compartments are arranged around the catheter in such a way that in the consecutive application of the included elements in chronological order of their linear sequence a succession of application steps is resulting that starts with wetting the catheter tip with lubricant, continues with taking the wetted catheter out of the tearable compartment, and finally comprises the insertion of an integrated quantum of irrigation liquid ready for application into the rectum.

2. Apparatus according to claim 1, **characterized by** the fact that the front part of the catheter in the area of its tip is located in a different compartment than its rear section.

3. Apparatus according to claim 1 or 2, **characterized by** the fact that in a compartment accommodating the catheter tip a gel cushion containing a wetting liquid is present.

4. Apparatus according to claim 1 through 3, **characterized by** the fact that a partition wall between the compartment accommodating the front part of the catheter and a compartment accommodating its rear section exhibits an opening throughout which the catheter extends, at which such opening is preferably sealed off against the catheter.

5. Apparatus according to one of the foregoing claims, **characterized by** the fact that at least one compartment initially accommodating the catheter or parts of the same is tearable along a ripping line, for example in form of a perforation line, to enable taking out the catheter.

6. Apparatus according to one of the foregoing claims, **characterized by** the fact that one or more compartments initially accommodating the catheter are connected to further sections or compartments of the foil structure (39) by means of predetermined breaking points, for instance in form of perforated lines, so that the shroud of the compartments initially accommodating the catheter is separable from the rest of the sections or compartments of the foil structure (39), in particular is rippable off from the later.

7. Apparatus according to one of the foregoing claims, **characterized by** the fact that one or more connection hoses are located inside a common compartment together with the catheter, particularly together with the rear section of the catheter.

8. Apparatus according to one of the foregoing claims, **characterized by** the fact that a connection hose leads to a liquid reservoir, in particular leads to a liquid reservoir located in a third compartment.

9. Apparatus according to one of the foregoing claims, **characterized by** the fact that the liquid reservoir is dimensioned in such a way that volume contained in it is sufficient for one irrigation process.

10. Apparatus according to one of the foregoing claims, **characterized by** the fact that one or more additional compartments filled with air or another gas are provided, which is used to inflate the balloon of the apparatus in order to fix the same inside a patient and/or to initiate the defecation process.

11. Apparatus according to one of the foregoing claims, **characterized by** the fact that one or more compartments filled with air or another gas are connected through a connection line to a balloon in or at the catheter for filling the later.

12. Apparatus according to one of the foregoing claims, **characterized by** the fact that a non-return valve is installed in or at the connection hose from an air, gas, or liquid reservoir or compartment to the catheter.

13. Apparatus according to one of the foregoing claims, **characterized by** the fact that a connection hose with a connection for a preferably external compressed air supply source is provided.

14. Apparatus according to one of the foregoing claims, **characterized by** the fact that the layers of foil of the foil structure (39) are welded airtight at their outer edges and/or along internal separating lines.

15. Apparatus according to one of the foregoing claims, **characterized by** the fact that one or more ripping lines, in particular perforation lines, are running on or along the weld lines, in particular in the areas of internal separation lines, where the foils are airtight welded together.

## Revendications

1. Dispositif pour l'introduction transanale d'un lavement dans le rectum/ dans le colon d'un patient, avec un ballon gonflable de la forme d'une haltère ou d'un sablier, placé par voie transanale, les extrémités du ballon étant positionnés sur le corps de la tige du cathéter de façon inversée afin que les parties des extrémités du ballon s'approchent après remplissage dans des sens opposés l'un de l'autre, en passant par la partie étroite au milieu, et que l'ouverture distale ou l'extrémité distale de la tige du cathéter ne dépasse pas en direction distale le zénith du rayon avant, de front, du ballon lors de la position au repos, non déviée du corps de la tige dans le ballon placé in situ par voie transanale, **marquée** par une structure de films (39) de couches soudées, de préférence soudées sous forme de sachet, avec plusieurs compartiments formés, dans laquelle sont emballés la tige du cathéter ainsi qu'un lubrifiant, les compartiments étant positionnés autour du cathéter de façon à ce qu'en cas de l'application successive des éléments contenus dans l'ordre chronologique, leur juxtaposition linéaire forme une séquence d'étapes d'applications, commençant par l'humectage de la pointe du cathéter avec le lubrifiant, continuant par le prélèvement du cathéter humecté du compartiment déchirable sous forme de sachet, et incluant ensuite l'introduction d'une quantité de liquide d'irrigation - intégrée dans le produit et prêt à l'emploi.

2. Dispositif selon revendication n° 1, distingué par le fait que la partie avant du cathéter se trouve dans un autre compartiment par rapport à la zone de sa pointe que le compartiment de sa zone arrière.

3. Dispositif selon revendication n° 1 ou 2 distingué par le fait que dans un compartiment logeant la pointe du cathéter se trouve un coussin de gel avec un liquide humectant.

4. Dispositif selon l'une des revendications n° 1 à 3, distingué par le fait qu'une paroi entre un compartiment logeant la partie avant du cathéter et un compartiment logeant la partie arrière dispose d'une ouverture par laquelle le cathéter passe, cette ouverture étant de préférence étanchéifiée par rapport au cathéter.

5. Dispositif selon l'une des revendications précédentes, distingué par le fait qu'au moins un compartiment logeant initialement le cathéter ou ses composants soit déchirable le long d'une ligne de déchirage, p.ex. sous forme d'une ligne perforée, permettant le prélèvement du cathéter.

6. Dispositif selon l'une des revendications précédentes, distingué par le fait qu'un ou plusieurs des compartiments logeant initialement le cathéter soient reliés avec d'autres sections ou compartiments de la structure de film (39) moyennant des points de rupture, p.ex. sous forme de lignes perforées ou similaires, de manière à ce que la housse des compartiments logeant initialement le cathéter soit séparable des autres sections ou compartiments de la structure de film (39), notamment qu'ils soient détachables par arrachement.

7. Dispositif selon l'une des revendications précédentes, distingué par le fait qu'un ou plusieurs tubes de connexion se trouvent dans le même compartiment que le cathéter, notamment la partie arrière du cathéter.

8. Dispositif selon l'une des revendications précédentes, distingué par le fait qu'un tube de connexion mène vers un réservoir de liquide, notamment vers un réservoir de liquide dans un troisième compartiment.

9. Dispositif selon l'une des revendications précédentes, distingué par le fait que le réservoir de liquide soit dimensionné assez grand pour que le contenu soit suffisant pour une procédure d'irrigation.

10. Dispositif selon l'une des revendications précédentes, distingué par le fait qu'un ou plusieurs autres compartiments remplis d'air ou d'un autre gaz soient prévus, permettant de gonfler un ballon du dispositif afin d'ancrer celui-ci dans un patient et/ ou de déclencher un réflexe de défécation.

11. Dispositif selon l'une des revendications précédentes, distingué par le fait qu'un ou plusieurs autres compartiments remplis d'air ou d'un autre gaz soient reliés par une conduite de connexion avec un ballon ou au cathéter pour le remplissage de celui-ci.

12. Dispositif selon l'une des revendications précédentes, distingué par le fait qu'une valve anti-retour soit disposée dans ou à un tube de connexion d'un réservoir ou compartiment d'air, de gaz ou de liquide vers le cathéter.

13. Dispositif selon l'une des revendications précédentes, distingué par le fait qu'un tube de connexion avec un raccord pour une source d'air comprimé - externe de préférence - soit prévu.

14. Dispositif selon l'une des revendications précédentes, distingué par le fait que des couches de film de la structure de film (39) soient soudées à leur bord extérieur et/ ou le long de lignes de séparation de façon étanche à l'air.

15. Dispositif selon l'une des revendications précédentes, distingué par le fait qu'une ou plusieurs lignes de déchirement - notamment des lignes de perforation - se trouvent sur ou le long de lignes de soudage, notamment dans une zone de lignes intérieures de séparation où les films sont joints par soudage.
